# EUROPEAN PATENT SPECIFICATION

(11) **EP 2 260 873 B1**
(45) Date of publication and mention of the grant of the patent: **24.07.2019**
(21) Application number: 09162203.5
(22) Date of filing: 08.06.2009
(51) Int. Cl.: A61K 47/58

(54) **PCYLATION OF PROTEINS**
PCYLIERUNG VON PROTEINEN
PCYLATION DE PROTÉINES

(43) Date of publication of application: 15.12.2010
(73) Proprietor: Biocompatibles UK Limited, Farnham Surrey GU9 8QL (GB)
(72) Inventor: Lewis, Andrew, Farnham, Surrey, GU9 8QL (GB)
(74) Representative: BTG plc Intellectual Property Group

(56) References cited:
- WO-A-2007/100902
- WO-A-2008/098930
- LEWIS A ET AL: "Poly(2-methacryloyloxyethyl phosphorylcholine) for protein conjugation" BIOCONJUGATE CHEMISTRY 200811 US, vol. 19, no. 11, November 2008 (2008-11), pages 2144-2155, XP002544347 ISSN: 1043-1802
- SAMANTA DEBASIS ET AL: "End-functionalized phosphorylcholine methacrylates and their use in protein conjugation." BIOMACROMOLECULES OCT 2008, vol. 9, no. 10, October 2008 (2008-10), pages 2891-2897, XP002544348 ISSN: 1526-4602
- SHAUNAK SUNIL ET AL: "Site-specific PEGylation of native disulfide bonds in therapeutic proteins." NATURE CHEMICAL BIOLOGY JUN 2006, vol. 2, no. 6, June 2006 (2006-06), pages 312-313, XP002544349 ISSN: 1552-4450
- LIBERATORE F A ET AL: "SITE-DIRECTED CHEMICAL MODIFICATION AND CROSS-LINKING OF A MONOCLONAL ANTIBODY USING EQUILIBRIUM TRANSFER ALKYLATING CROSS-LINK REAGENTS" BIOCONJUGATE CHEMISTRY, ACS, WASHINGTON, DC, US, vol. 1, no. 1, 1 January 1990 (1990-01-01), pages 36-50, XP002313939 ISSN: 1043-1802

## Description

### Field of Invention

This invention relates to the modification of proteins with polymers for the purpose of improving their efficacy as therapeutic treatments.

### Background to Invention

In the past, therapeutic proteins have had several inherent shortcomings. Proteins often have short half-lives, wide tissue distribution, the potential for immunogenicity, and sometimes need to be dosed frequently. When frequent dosing is required, it can result in increased cost, toxicity and complicated dosing regimens. In an effort to overcome these shortcomings, researchers have looked at improving the delivery systems of proteins. Among the potential solutions lies PEGylation, which involves the attachment of a flexible strand or strands of polyethylene glycol (PEG) to a protein. The size, weight, shape and the linkage used to connect the PEG strand to the protein determine the impact of PEGylation on absorption, volume of distribution, proteolysis, circulation lifetime, renal clearance and toxicity of the protein.

When attached to a drug or protein, PEG polymer chains can sustain bioavailability by protecting the drug molecules from immune responses and other clearance mechanisms in the body. In an aqueous medium, the long chain-like PEG molecule is heavily hydrated and in rapid motion. This motion is believed to cause the PEG molecule to sweep out a large volume and prevent the interference of other molecules.

PEGylation is used in tumour targeting and diagnostic imaging applications. PEGylated liposomes, for instance, are employed in several marketed formulations. PEGylation of surfaces is an efficient method to enhance the biocompatibility of a synthetic material. In drug development, PEGylation is an established method to improve on the pharmacokinetic profile of therapeutic compounds. PEGylation has been very successfully applied to the development of second-generation biotherapeutics and captured a significant portion of the US biologicals market.

The numerous advantages of PEGylation make this one of the most powerful technologies for the engineering of biotherapeutics. Clinical developments are reported for PEG conjugates of proteins, peptides, aptamers, natural products and small molecules.

L-asparaginase (denoted asparaginase) is an enzyme that catalyzes the hydrolysis of asparagine to aspartic acid. A bacterially-derived asparaginase is marketed under the brand name ElsparR™ (Merck & Co) to treat acute lymphoblastic leukaemia and lymphomas. A PEGylated version is marketed under the brand name Oncaspar™. Leukemic cells depend on circulating asparagine, and treatment with asparaginase deprives these cells of this nutrient essential for their growth and replication. Approximately a third of all patients receiving the treatment eventually develop blocking antibodies against the native *E. coli* asparaginase, and are unable to continue with treatment. A major concern with this treatment is the immunogenicity of asparaginase produced by *E.coli,* which can lead to anaphylactic reactions and early clearance by serum antibodies. The PEGylated form of asparaginase is less immunogenic and as a result the development of antibodies can be substantially attenuated.

*E. coli* derived asparaginase is a protein (MW 133-138 kDa) that consists of four identical subunits, each of which contains 326 amino acids and a single disulfide bond between Cys77-Cys105. Asparaginase must exist in its tetrameric form to display its biological activity. Structurally, the disulfides are accessible because they lie on the outer edge of the protein and are distant from the protein's enzymatic site. L-asparaginase has been shown to undergo disulfide bond PEGylation (Balan et al. (2007) Site-Specific PEGylation of Protein Disulfide Bonds Using a Three-Carbon Bridge. Bioconjugate Chem 18:61-76). In an enzyme immunoassay (ELISA) that was based upon the use of a rabbit polyclonal anti-asparaginase antibody, the *in vitro* antigenicity of the disulfide bond PEGylated asparaginase was similar to that of the native asparaginase. These results are consistent with the site-selective conjugation of PEG to a site that is distant to the enzyme's antigenic site. Disulfide bond PEGylated asparaginase retained its enzymatic activity and its antigenicity, irrespective of the size of PEG attached. In contrast, commercially available amine bond PEGylated asparaginase has lower enzymatic activity and is less antigenic. This is thought to be due to the hyper-PEGylation that characterises the commercially available PEG-asparaginase which has approximately 40 molecules of 5 kDa PEGs per protein tetramer. There is therefore scope to improve this therapeutic, and indeed other therapeutic proteins also, by conjugation with a polymer that reduces immunogenicity whilst maintaining high levels of protein activity.

### Summary of Invention

Herein is disclosed a polymer comprising 2-methacryloyloxyethyl-phosphoryl chlorine (MPC) for use in reducing the immunogenicity exhibited by lower antibody titres of a therapeutic protein which is administered to the human or animal body, wherein a covalently-linked conjugate of the polymer and therapeutic protein is administered.

The invention provides the use of a polymer comprising MPC as defined in the claims polymers comprising MPC of controlled architecture may be made using methods known in the art such as atom transfer radical polymerisation (ATRP) or radical addition-fragmentation chain transfer polymerisation (RAFT), for instance as described in US6,852,816. We have previously described methods of attaching polymers to proteins *via* conventional linking technologies described in the art (WO2004/063237) or by modification of the protein into an initiator for polymerisation of said polymers (WO 03/062290).

The polymers, based on phosphorylcholine (PC) are functionalised with an end-terminal linking group capable of insertion into the disulfide bridges or reaction with terminal cysteine residues of many commonly used therapeutic proteins. This technology is described in detail in WO2008/098930. The resulting conjugate has reduced immunogenicity by virtue of the presence of the MPC residues which are effective at reducing antibody recognition of the protein. Advantageously, site-specific attachment away from the active site of the protein allows for retention of significant activity.

It will be noted that WO2008/098930 discloses that MPC polymer provides improved protein shielding from antibodies. However, the present invention is based on the finding that conjugating PC polymer to therapeutic proteins can allow the proteins to avoid detection by the immune system, thus leading to low antibody titres *in vivo* upon exposure of the protein in the bloodstream.

Antigen presentation is a process in the body's immune system by which macrophages, dendritic cells and other cell types capture antigens and the enable their recognition by T-cells. Activation of T-cells ultimately leads to antibody production. PCylation (the process of conjugating with PC) provides an effective method of camouflaging the underlying antigen regions of the therapeutic proteins, preventing recognition and engulfment by Antigen Presenting Cells or binding to the surface antibodies presented on B cells, thus inhibiting the production of further antibodies. Hence, in this way PCylation of a protein seeks to avoid the immune response by blocking the early recognition pathways that would otherwise result in antibody production. This is distinctly different to the finding that MPC polymer provides improved protein shielding from antibodies, which relies on PCylation to act as a steric barrier to shield the protein from antibodies directed towards the protein.

### Detailed Description of Invention

The polymer used in this invention comprises MPC. The polymer is to be administered in the form of a covalently-linked conjugate of the polymer and a therapeutic protein. Typically, the protein comprises one or more cysteine residues and the protein is covalently linked to the polymer via one or more of said cysteine residues. In a particularly preferred embodiment, the polymer has formula I or II.: 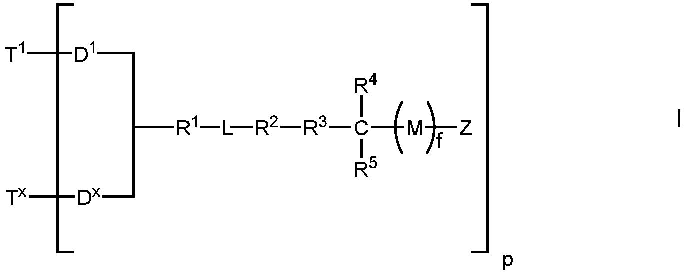 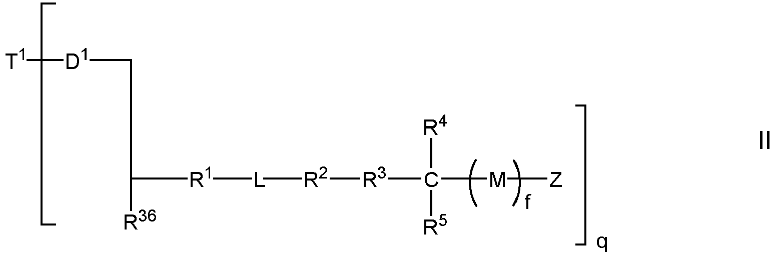
wherein R¹ is an electron-withdrawing group;
R² is selected from C=O, C(=O)NR⁹ or a bond; wherein R⁹ is H or C₁₋₄ alkyl;
R³ is selected from the group consisting of C₁-C₂₀ alkylene, C₃-C₈ cycloalkylene, C(=O)R¹⁰, C(=O)NR¹¹, C₁-C₂₀ alkoxy, C₂-C₂₀ alkenylene, C₂-C₂₀ alkenyl oxiranylene, arylene, heterocyclene and aralkylene; in which 0 to all of the hydrogen atoms are replaced with halogen, C₁-C₆ alkyl optionally substituted with 1 to 2 substituents selected from the group consisting of C₁-C₄ alkoxy, aryl, heterocyclyl, C(=O)R¹³, C(=O)NR¹¹R¹², oxiranyl and glycidyl;
R¹⁰ is alkylene of from 1 to 20 carbon atoms, alkoxy from 1 to 20 carbon atoms, oligo(alkoxy) in which each alkoxy group has from 1 to 3 carbon atoms, aryloxy or heterocyclyloxy; any of which groups may have substituents selected from optionally substituted alkoxy, oligoalkoxy, amino (including mono- and di-alkyl amino and trialkyl ammonium, which alkyl groups, in turn, may have substituents selected from acyl, alkoxycarbonyl, alkenoxycarbonyl, aryl and hydroxy) and hydroxyl groups;
R¹¹ and R¹² are independently H or alkyl of from 1 to 20 carbon atoms, or R¹¹ and R¹² may be joined together to form an alkanediyl group of from 2 to 5 carbon atoms, thus forming a 3- to 6- membered ring;
R⁴ and R⁵ are each independently selected from H, Z, halogen, C₁₋₂₀ alkyl, C₃-C₈ cycloalkyl, OH, CN, C₂-C₂₀ alkenyl, C₂-C₂₀ alkenyl oxiranyl, C(=O)R¹³, glycidyl, aryl, heterocyclyl, arylkyl, aralkenyl, in which 0 to all of the hydrogen atoms are replaced with halogen, C₁-C₆ alkyl optionally substituted with 1 to 2 substituents selected from the group consisting of C₁-C₄ alkoxy, aryl, heterocyclyl, C(=O)R¹³, C(=O)NR¹¹R¹², oxiranyl and glycidyl;
where R¹³ is alkyl of from 1 to 20 carbon atoms, alkoxy of from 1 to 20 carbon atoms, oligo(alkoxy) in which each alkoxy group has 1 to 3 carbon atoms, aryloxy or heterocyclyloxy any of which groups may have substituents selected from optionally substituted alkoxy, oligoalkoxy, amino (including mono- and di-alkyl amino and trialkyl ammonium, which alkyl groups, in turn may have substituents selected from acyl, alkoxycarbonyl, alkenoxycarbonyl, aryl and hydroxy) and hydroxyl groups; and
L is a linking group;
Groups M are the same or different and are residues of ethylenically unsaturated monomers, wherein at least one of the residues M is MPC;
f is 1 to 500;
R³⁶ is -CH₂E⁴, H or C₁₋₄ alkyl; and
E⁴ is a leaving group;
x is 1 or 2;
p is 1-5 and q is 1-10;
D is S, NH or O; and
T-D is a therapeutic protein; wherein T¹D¹ and T²D² (if present) are different biological molecules.

The ethylenically unsaturated monomers are preferably polymerised by a living radical polymerisation process in the presence of an initiator of the general formula III or IV and a catalyst 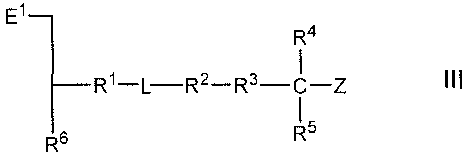 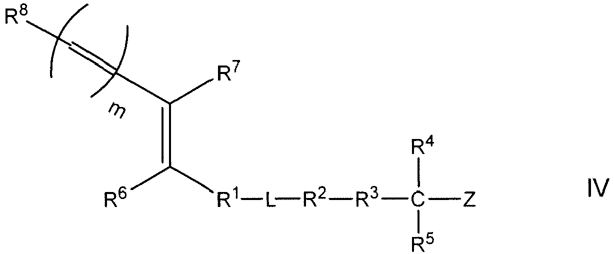
wherein R¹ is an electron-withdrawing group;
R² is selected from C=O, C(=O)NR⁹ or a bond;
wherein R⁹ is H or C₁₋₄ alkyl;
R³ is selected from the group consisting of C₁-C₂₀ alkylene, C₃-C₈ cycloalkylene, C(=O)R¹⁰, C(=O)NR¹¹, C₁-C₂₀ alkoxy, C₂-C₂₀ alkenylene, C₂-C₂₀ alkenyl oxiranylene, arylene, heterocyclene and aralkylene; in which 0 to all of the hydrogen atoms are replaced with halogen, C₁-C₆ alkyl optionally substituted with 1 to 2 substituents selected from the group consisting of C₁-C₄ alkoxy, aryl, heterocyclyl, C(=O)R¹³, C(=O)NR¹¹R¹², oxiranyl and glycidyl;
R¹⁰ is alkylene of from 1 to 20 carbon atoms, alkoxy from 1 to 20 carbon atoms, oligo(alkoxy) in which each alkoxy group has from 1 to 3 carbon atoms, aryloxy or heterocyclyloxy; any of which groups may have substituents selected from optionally substituted alkoxy, oligoalkoxy, amino (including mono- and di-alkyl amino and trialkyl ammonium, which alkyl groups, in turn, may have substituents selected from acyl, alkoxycarbonyl, alkenoxycarbonyl, aryl and hydroxy) and hydroxyl groups;
R¹¹ and R¹² are independently H or alkyl of from 1 to 20 carbon atoms, or R¹¹ and R¹² may be joined together to form an alkanediyl group of from 2 to 5 carbon atoms, thus forming a 3- to 6- membered ring;
R⁴ and R⁵ are each independently selected from H, Z, halogen, C₁₋₂₀ alkyl, C₃-C₈ cycloalkyl, OH, CN, C₂-C₂₀ alkenyl, C₂-C₂₀ alkenyl oxiranyl, C(=O)R¹³, glycidyl, aryl, heterocyclyl, arylkyl, aralkenyl, in which 0 to all of the hydrogen atoms are replaced with halogen, C₁-C₆ alkyl optionally substituted with 1 to 2 substituents selected from the group consisting of C₁-C₄ alkoxy, aryl, heterocyclyl, C(=O)R¹³, C(=O)NR¹¹R¹², oxiranyl and glycidyl;
where R¹³ is alkyl of from 1 to 20 carbon atoms, alkoxy of from 1 to 20 carbon atoms, oligo(alkoxy) in which each alkoxy group has 1 to 3 carbon atoms, aryloxy or heterocyclyloxy any of which groups may have substituents selected from optionally substituted alkoxy, oligoalkoxy, amino (including mono- and di-alkyl amino and trialkyl ammonium, which alkyl groups, in turn may have substituents selected from acyl, alkoxycarbonyl, alkenoxycarbonyl, aryl and hydroxy) and hydroxyl groups; and
L is a linking group;
Z is selected from the group consisting of Cl, Br, I, OR¹⁴, SR¹⁵, SeR¹⁵, OP(=O)R¹⁵, OP(=O)(=OR¹⁵)₂, O-N(R¹⁵)₂ and S-C(=S)N(R¹⁵)₂, where R¹⁴ is alkyl of from 1 to 20 carbon atoms in which each of the hydrogen atoms may be independently replaced by halide, R¹⁵ is aryl or a straight or branched C₁-C₂₀ alkyl group, and where an N(R¹⁵)₂ group is present, the two R¹⁵ groups may be joined to form a 5- or 6- membered heterocyclic ring;
R⁶ is CH₂E², H or C₁₋₄ alkyl;
R⁷ and R⁸ are each independently selected from H and C₁₋₄ alkyl;
m is 0-4; and
E¹ and E² are each, independently, a leaving group, or a precursor to a leaving group.

A polymer of general formula V or VI may be formed in this method: 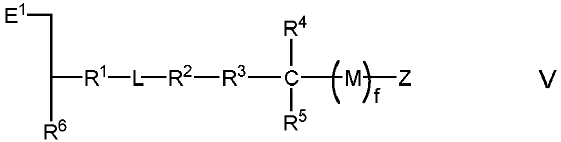 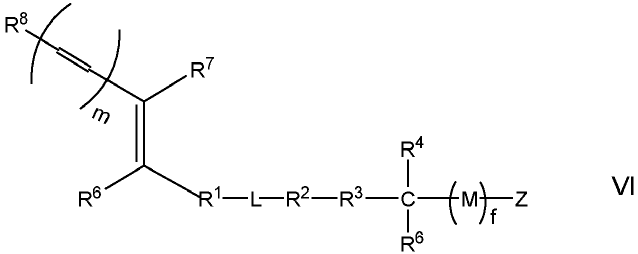 wherein R¹-R⁶, R⁷-R⁸ (if present), L, Z, m, and E¹ are as defined above:
f is 1 to 500; and
Groups M are the same or different and are residues of ethylenically unsaturated monomers. At least one group M is a residue of MPC.

These polymers are used to derivatise therapeutic proteins. A method of derivatisation of a biological molecule may be used in which a polymer of general formula V or general formula VI is added to a therapeutic protein of general formula T¹D¹X or to therapeutic proteins of general formula T¹D¹X and T²D²X, wherein D is S, NH or O and X is H, D¹T¹ or D²T² to form an adduct of general formula VII or of general formula VIII 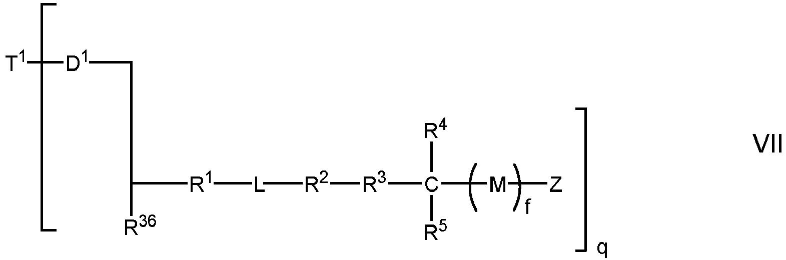 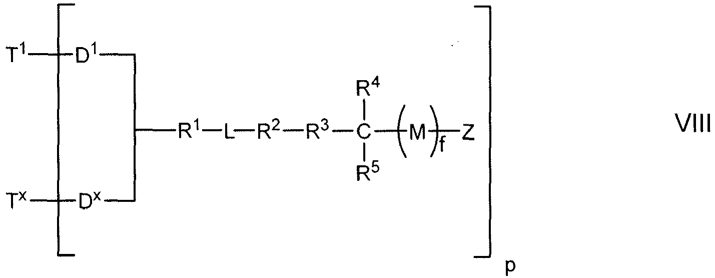 wherein
R¹-R⁵, R⁷-R⁸ (if present), m and L are as defined in the first aspect of the invention and M is as defined above in the third aspect of the invention;
f is 1 to 500;
R³⁶ is -CH₂E⁴, H or C₁₋₄ alkyl; and
E⁴ is a leaving group;
x is 1 or 2;
p is 1-5 and q is 1-10.

Compounds of general formulae III and IV act as initiators in the living radical polymerisation process. The initiators are extended at the carbon to which Z is attached by a living radical polymerisation process. The opposite end of the initiator molecule must be suitable for derivatisation with a therapeutic protein. In this regard it is vital that the initiator has an α-methylene leaving group, or precursor to a leaving group, or a double bond conjugated with an electron-withdrawing group. This allows a biological molecule to react with the initiator in a Michael reaction.

When referring to groups which are radicals, it is intended that the group can be joined to adjacent groups in either direction. For instance when R³ is C(=O)R¹⁰, it may be linked either R²-C(=O)R¹⁰-C or R²-R¹⁰C(=O)-C.

Preferably, in compounds of formula III and IV, R⁶ is -CH₂E². If the leaving group is prone to elimination rather than to direct displacement with a nucleophile, and the electron-withdrawing group R¹ is a suitable activating moiety for the Michael reaction then sequential intramolecular bis-alkylation can occur by consecutive Michael and retro Michael reactions. The leaving moiety serves to mask a latent conjugated double bond that is not exposed until after the first alkylation has occurred and bis-alkylation results from sequential Michael and retro-Michael reactions.

Initiators of general formula IV may have from 1 to 5 double bonds. A nucleophile may add to the conjugated system at a suitable position on any of the double bonds. Preferably, however, m is zero in an initiator of general formula II.

Preferably, in the initiators of general formula III and IV, R⁶, R⁷ and R⁸ are each independently selected from -H or -CH₃.

In the initiator of general formula III or IV it is preferred that only one of R⁴ and R⁵ is H. Preferably, neither are hydrogen. Suitably, at least one, and preferably both of R⁴ and R⁵ are methyl.

In a preferred embodiment, R³ is -CO-R¹⁰ in which R¹⁰ is oligoalkoxy, preferably oligoethoxy in which there are 2 to 10 ethoxy groups. Alternatively, the alkoxy group may have 1 or 3 carbon atoms. R⁴ and R⁵ are preferably methyl.

Group Z is a radically transferable group and is preferably a halogen, more preferably Cl, Br or I, most preferably Br. Since groups R⁴ and R⁵ may also be Z the initiator may be di-, oligo- or poly- functional.

Suitable linking groups, L, include a bond, a C₁₋₁₀ alkylene group, or an optionally substituted aryl or heteroaryl, any of which groups may have substituents selected from optionally substituted alkoxy, oligoalkoxy, amino (including mono- and di-alkyl amino and trialkyl ammonium, which alkyl groups, in turn, may have substituents selected from acyl, alkoxycarbonyl, alkenoxycarbonyl, aryl and hydroxy) and hydroxyl groups.

Suitable groups for electron-withdrawing group R¹ include a keto group, an ester group, and a sulphone group.

Groups E¹ and E² are typically leaving groups selected from -SR¹⁷,-SO₂R¹⁷, -OSO₂R¹⁷, -N⁺R¹⁷₃, -N⁺HR₂¹⁷, -N⁺H₂R¹⁷ halogen, or -OAr, in which R¹⁷ represents an alkyl or aryl group and Ar represents a substituted aryl group containing at least one electron-withdrawing substituent.

Alternatively, groups E¹ and E² may be precursors to leaving groups, wherein the leaving group may be obtained by a simple chemical reaction such as oxidation or reduction. Having E¹ and E² as precursors to leaving groups may be preferable if this facilitates synthesis of the initiator and the subsequent polymerisation reaction.

In a preferred embodiment of this invention, E¹ and E² are each, independently, an (optionally substituted) aryl sulfide or aryl sulfone group.

An aryl sulfide (a precursor to a leaving group) may be converted into an aryl sulfone (a leaving group) by a simple oxidation reaction, as is well known in the art. The process may include a subsequent reaction step in which groups E¹ and E² (if present) which are precursors to leaving groups are converted into leaving groups.

A particularly preferred initiator of general formula III has formula:

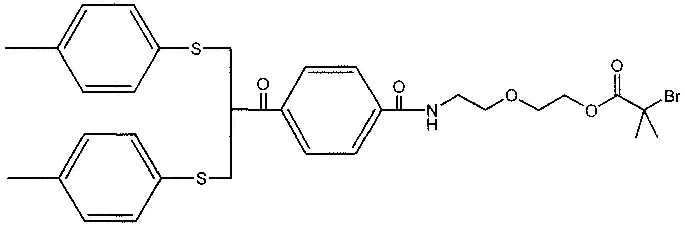

The living radical polymerisation process used in the invention may be a group transfer radical polymerisation, for instance in which an N-O, or other carbon-, sulphur-, and oxygen- centred radical group is transferred from an initiator compound to a monomer. Preferably, however, the process is an atom transfer radical polymerisation process.

The polymers used in this invention are generally made by a living radical polymerisation process. Other controlled polymerisation techniques may be used for instance NO group transfer systems such as are described in WO-A-0018807, catalyst systems described in WO-A-9958588, systems involving irradiation with visible light, or other EM radiation such as described in WO-A-99/10387, radical addition fragmentation chain transfer polymerisation (RAFT) as described in Rizzardo, E. et al. ACS Symposium Series 2000, 768, 278-296, using compounds (initiators) of the general type Z-C=SSR or macromolecular design through interchange of xanthes (MADIX) as descried by Bontevin, B., J. Polym.Sci. PtA, Polym.Chem., 2000, 38(18), 3235-3243.

Atom or group transfer radical polymerisation processes are described further in WO 02/28929 and WO 03/074090; pages 15-24.

The living radical polymerisation process used in the invention is preferably carried out to achieve a degree of polymerisation in the range 5 to 500. Preferably the degree of polymerisation is in the range 10 to 100, more preferably in the range 10 to 50. In the preferred group or atom transfer radical polymerisation technique, the degree of polymerisation is directly related to the initial ratios of initiator to monomer. Preferably the ratio is in the range 1:(5 to 500), more preferably in the range of 1:(10 to 100), most preferably in the range 1:(10 to 50).

At least one of the ethylenically unsaturated monomers is MPC. The others may be anionic, cationic or nonionic monomers.

The polymer may be a homopolymer that is moiety -(-M-)-_{f} may comprise groups M which are residues of the same ethylenically unsaturated monomer MPC. Alternatively, the polymer may be a copolymer or a block copolymer, wherein groups M in moiety -(-M-)-_{f} are residues of different ethylenically unsaturated monomers.

Preferably, the ethylenically unsaturated comonomers are zwitterionic. The zwitterionic nature arises from the combination of a cationic and anionic moiety. The cationic moiety may be an ammonium, phosphonium or sulphonium group, preferably an ammonium group. The anion is typically a phospho moiety, more typically a phosphate diester.

In polymers of general formula I and II, the preferred groups L, R¹-R⁶ and Z are the same as outlined above. For the polymers to be able to conjugate therapeutic proteins, groups E¹ and E² (if present) in the polymers of formula V and VI should be leaving groups. Accordingly, a particularly preferred leaving group is:

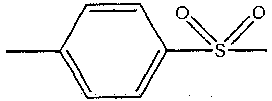

Particularly preferred polymers which have formed covalently-linked conjugates of polymer and therapeutic protein are of formula: 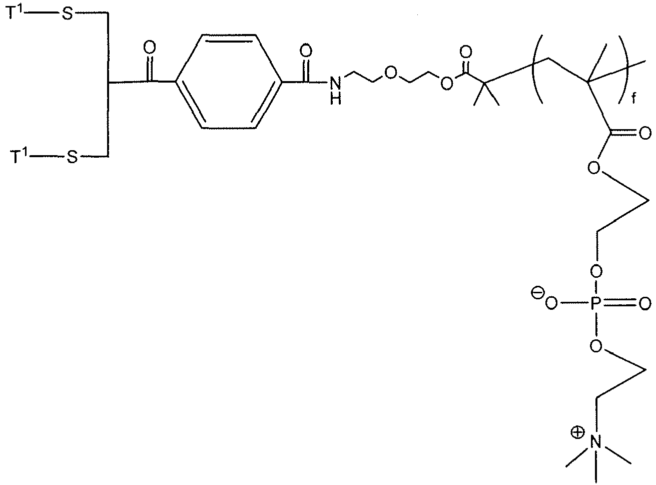 and

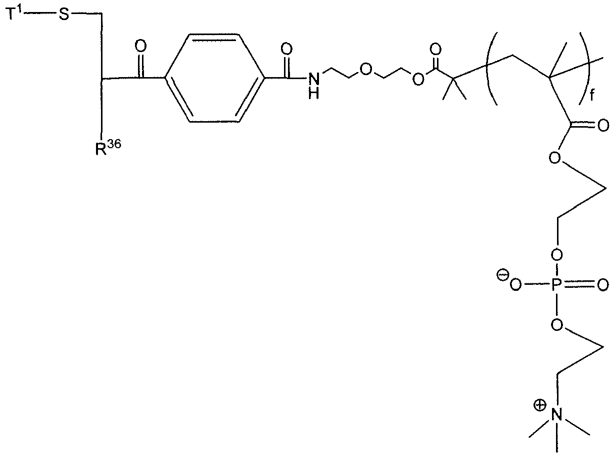

The therapeutic protein or proteins are represented by general formulae T¹D¹X and T²D²X wherein D¹X and D²X represent a nucleophilic group. Typically, the nucleophilic group is a thiol group, although amine and hydroxyl groups may also be used to conjugate the therapeutic protein to the MPC polymer.

An adduct of general formula II is formed when the polymer of general formula V or VI has only one leaving group, or R⁶ is not a leaving group in a polymer of general formula V. Adducts of general formula II may form when a nucleophile attacks the polymer of general formula V or VI twice, i.e. when there are two leaving groups (E¹ and E²), or R⁶ is a leaving group in V.

Moiety T^{x}-D^{x}- in formula I may be derived from the same therapeutic protein molecule as T¹-D¹, in which case x is 1. Alternatively, moiety T^{x}-D^{x}- may be derived from a different therapeutic protein, in which case x is 2.

Typically, the derivatisation will be carried out by partially reducing a disulphide bond derived from two cysteine amino acids in the same protein. Since in this case T¹D¹ and T^{x}D^{x} are derived from the same protein, x = 1.

Suitably, the process utilised in the invention is carried out by partially reducing a disulfide bond derived from two cysteine amino acids in the protein *in situ* following which the reduced product reacts with the polymer of formula V or VI. Disulfides can be reduced, for example, with dithiothreitiol, mercaptoethanol, or tris-carboxyethylphosphone using conventional methods. The process may be carried out in a solvent or solvent mixture in which all reactants are soluble. The protein containing nucleophilic groups may be allowed to react directly with the polymer of the general formula V or VI in an aqueous reaction medium. This reaction medium may also be buffered, depending on the pH requirements of the nucleophile. The optimum pH for the reaction is generally between about 5.5 and about 8, for example 7.4, preferably about 6.0-6.5. Reaction temperatures between 3-37°C are generally suitable: proteins and other biological molecules may decompose or denature impairing function if the conjugation reaction is conducted at a temperature where these processes may occur. Reactions conducted in organic media (for example THF, ethyl acetate, acetone) are typically conducted at temperatures up to ambient, for example temperatures below 0°C.

A protein can contain one or a multiplicity of disulfide bridges. Reduction to give free sulfhydral moieties can be conducted to reduce one or a multiplicity of disulfide bridges in a protein. Depending on the extent of disulfide reduction in the stoichiometry of the polymeric conjugation reagent that is used, it is possible to conjugate one or a multiplicity of polymer molecules to the protein. Immobilised reducing agents may be used if it is desired to reduce less than the total number of disulfides, as can partial reduction using different reaction conditions or the addition of denaturants.

Alternatively the source of the thiol groups can be from cysteines or thiols not originally derived from a disulfide bridge. If the source of the thiol groups is a disulfide bridge, this may be intrachain or interchain.

The therapeutic protein can be effectively conjugated with polymers using a stoichiometric equivalent or a slight excess of polymer, unlike many prior art reagents. However, since the polymers do not undergo competitive reactions with aqueous media used to solvate proteins, it is possible to conduct conjugation reaction with an excess stoichiometry of polymer. The excess polymer can be easily removed by ion exchange chromatography during routine purification of proteins.

The therapeutic protein may be, for example, a polypeptide, antibody, antibody fragment, enzyme, cytokine, chemokine or receptor. Constrained or cyclic polypeptides, which are usually cyclised through a disulphide bridge, and epitopes, may also be used.

Preferably, the therapeutic protein is an enzyme. Enzymes include carbohydrate-specific enzymes, proteolytic enzymes and the like. Enzymes of interest, for both industrial (organic based reactions) and biological applications in general and therapeutic applications in particular include the oxidoreductases, transferases, hydrolases, lyases, isomerases and ligases disclosed by US 4,179,337. Specific enzymes of interest include asparaginase, arginase, adenosine deaminase, superoxide dismutase, catalase, chymotrypsin, lipase, uricase, bilirubin oxidase, glucose oxidase, glucuronidase, galactosidase, glucocerbrosidase, and glutaminase.

The therapeutic protein conjugated in the present invention may be, for example, factor 8, insulin, ACTH, glucagen, somatostatin, somatotropins, thymosin, parathyroid hormone, pigmentary hormones, somatomedins, erythropoietin, luteinizing hormone, hypothalamic releasing factors, antidiuretic hormones, prolactin, interleukins, interferons, colony stimulating factors, hemoglobin, cytokines, antibodies, glycopolypeptides such as immunoglobulins, ovalbumin, lipase, glucocerebrosidase, lectins, tissue plasminogen activator and glycosilated inerleukins, interferons and colony stimulating factors are of interest, as are immunoglobulins such as IgG, IgE, IgM, IgA, IgD and fragments thereof. The therapeutic protein is preferably a peptide hormone.

Preferably, the therapeutic protein is asparaginase.

MPC polymer in this invention is shown to reduce the immunogenecity of a therapeutic protein. Reduced immunogenicity is determined in a standard assay by measuring the antibody titres raised to the therapeutic protein with and without conjugated MPC polymer. Immunogenicity is generally measured using an IgG ELISA assay. Example 4 gives more details on the use of this assay.

The MPC polymer and therapeutic protein are generally administered to the human or animal body by direct injection. Typical pharmaceutical formulations comprise the MPC polymer and therapeutic protein the form of a conjugate together with one or more pharmaceutically acceptable excipients, which are selected from those known in the art.

The invention will now be illustrated by the following Examples, which refer to Figures 1-5, in which
Figure 1 is a Western Blot Analysis of MPC-IFN (A) and PEG-IFN (B);
Figure 2 is a chromatogram showing excess MPC polymer removal on a anion exchange column (1 ml HiTrap Q FF column from GS Healthcare);
Figure 3 shows the SDS-PAGE analysis of asparaginase-MPC reaction solutions showing oxidized asparaginase monomer A and MPCylated asparaginase monomer B (reaction time 4 h at room temperature V4-12% Bis-Tris, MOPS buffer & Novex Sharp markers from Invitrogen; Instant Blue stain from Expedeon).
Figure 4 shows the SDS-PAGE analysis of the PMPC-asparaginase sample used for the in vitro and in vivo studies; and
Figure 5 shows the mean α-Asp IgG titre pooled from five mice inoculated with asparaginase (Asp), MPC-asparaginase (MPC-Asp) or PBS in Asp-coated ELISA.

### Example 1: Reduced Antibody Staining for MPCylated Interferon (MPC-IFN).

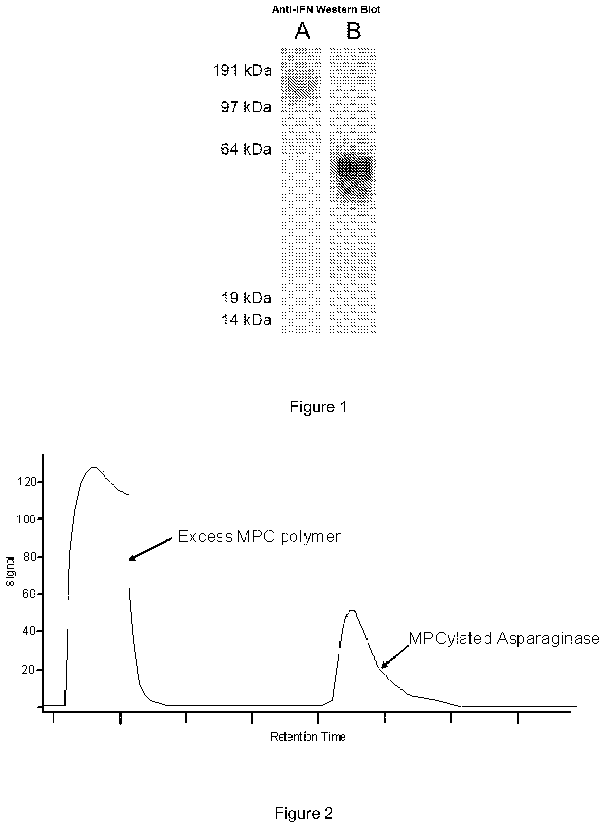

The preparation of MPC-IFN and corresponding PEG-IFN is described in detail in the Examples of WO2008/098930. 20kD PEG-IFN and a comparative MPC-IFN (20 kD equivalent on SEC, actual 40 kD) prepared using the same bis-sulphone linker were subjected to Western blot analysis using a polyclonal antibody against IFN alpha. Optimisation of antibody dilution and the length of incubation were required to improve the detection of MPC-IFN. PEGylation is known to cause steric shielding and consequently affects the way an antibody binds to a PEGylated protein. One aim of Western blot analysis was to assess the extent of steric shielding caused by MPCylation in comparison to PEGylation. When equal loadings of PEG-IFN and MPC-IFN were analysed (as determined by the height of the SEC trace peak at specific time points), no bands were detected for MPC-IFN (data not shown). To facilitate the detection of PC-IFN bands, larger volumes of PC-IFN were required, together with a longer incubation protocol (48 h incubation with 1:10,000 anti-IFN primary antibody). Finally, the MPC conjugate could be detected (Figure 1, lane A) but the intensity of the band was significantly lower for that of PEG-IFN (Figure 1, lane B) indicating the MPC was protecting the interferon from antibody recognition more efficiently.

### Example 2: Disulfide Bond MPCylation of Asparaginase

Active asparaginase is a tetramer with each monomer unit containing one disulfide bond. MPCylation of the disulfide bonds therefore allows up to four MPC polymer chains per active asparaginase tetramer depending on the efficiency of the MPCylation. Asparaginase from Escherichia coli was purchased from Sigma-Aldrich Ltd (product number A3809, lot number 117K4159). Poly(2-methacryloyloxyethyl phosphorylchloline) (PMPC or MPC polymer) with the bis-sulfide end-group was made as described in WO2008/098930. The end group was oxidised to the protein reactive bis-sulfone form as follows: MPC bis-sulfide (0.15 mg) was oxidised to the bis-sulfone end group using Oxone (7 mg) in a 1:1 v/v methanol:water mixture 2 ml) overnight. The MPCylation of asparaginase for *in vitro* and *in vivo* analysis was performed on asparaginase (4 mg, 1 mg/ml) reduced with DTT (100 mM) according to a revised procedure of Shaunak et al; Nat Chem Biol. 2006 Jun;2(6):312-3. MPC bis-sulfone (96 mg, molar excess) was allowed to react with the reduced asparaginase in pH 8.2 phosphate buffered saline (PBS) overnight at 4 °C. Excess MPC polymer was then removed using anion exchange chromatography with a 1 ml HiTrap Q FF column (GE Healthcare) at pH 8.0 (Figure 2).

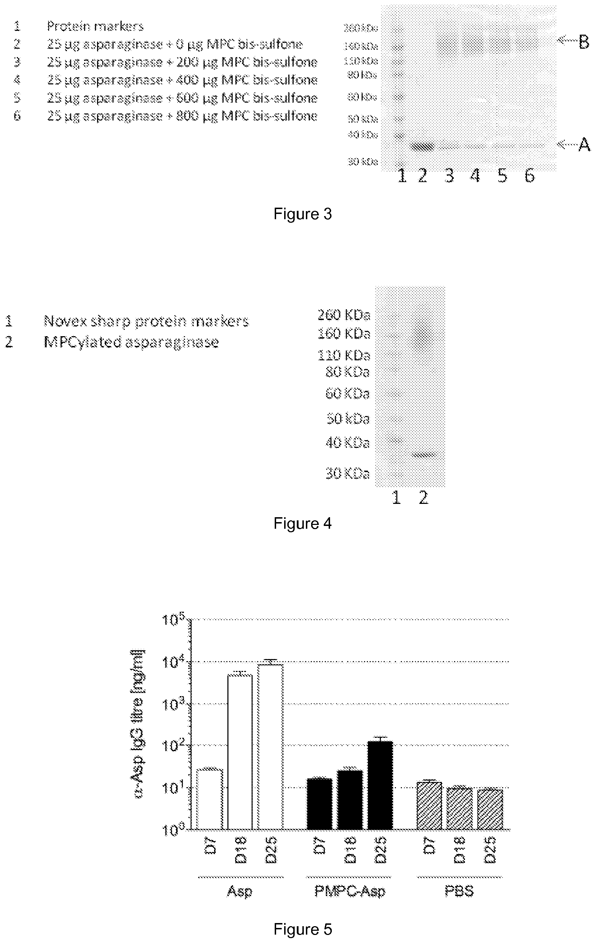

Reaction solutions during optimisation and scale-up were analysed by SDS-PAGE. The presence of SDS causes the asparaginase tetramer to dissociate into its monomeric constituents. The SDS-PAGE analysis revealed that it was possible to significantly MPCylate asparaginase (see Figure 3). It was not possible to convert all the four disulfide bonds of asparaginase to the MPCylated form as some non-MPCylated monomers were still detected (marked A in Figure 3). Increasing the amount of conjugating MPC polymer to the reaction did not appear to increase the conversion of monomer to MPCylated monomer (Lanes 3 to 6 in Figure 3). One explanation is that already conjugated MPC polymer sterically hinders the conjugation of further MPC polymer chains preventing full MPCylation in most cases. A second explanation or additional factor could be that the MPC polymer used causes reoxidation of the disulfide reduced protein due to oxidising metal ion contaminants such as copper. EDTA (20 mM) was added to the reaction buffer to minimise this effect.

The sample of MPCylated asparaginase used for the *in vitro* and *in vivo* studies is shown analysed by SDS-PAGE in Figure 4. The result shows significant MPCylation of the asparaginase tetramer with MPCylated monomer present around the 160 kDa protein marker (4-12% Bis-Tris gel and MOPS buffer from Invitrogen).

### Example 3: In vitro enzymatic activity of MPCylated asparaginase

Asparaginase is an amidase that converts L-asparagine to L-aspartic acid and ammonia. The most commonly used assay for asparaginase is based on the colorimetric determination of liberated ammonia from L-asparagine using the Nessler's reagent. Although the immunogenicity study does not require an *in vitro* validation of the PMPC-asparaginase, it was deemed necessary in order to ensure that the mice are immunised with a functional protein. Briefly, the amount of ammonia released when asparagine is incubated with PC-asparaginase is measured by comparing to a standard curve using a spectrophotometric determination method. The results are shown in Table 1.

| Table 1: *In vitro* activity of the native and MPCylated asparaginase. | | | |
|---|---|---|---|
| Sample | Conc (mg/ml) | NH₃ liberated (mmol) | Units/mg |
| Asparaginase | 0.180 | 4.2 | 173† |
| PMPC-asparaginase | 0.180 | 4.5 | 184 |
| †Manufacturer quoted activity is 129.6 units/mg | | | |

The conclusion is that MPCylation does not affect the enzymatic activity of asparaginase in an *in vitro* assay, and the PMPC-asparaginase was a suitable candidate for the subsequent *in vivo* immunogenicity study.

### Example 4: In vivo immunogenicity of MPCylated asparaginase

Determination of the immunogenic potential of MPCylated asparaginase was performed by repeated immunisation of the native non-MPCylated asparaginase and the MPCylated asparaginase separately in mice.

### Detection of anti-asparaginase antibody in mouse plasma

The amount of anti-asparaginase (a-Asp) antibody was measured using a custom ELISA. Briefly, microtitre plates designed for ELISA (Maxisorp, Nunc) were coated with 50 µl of native or MPCylated asparaginase (2 µg/ml in binding buffer) and incubated for 18 h at 4°C. After washing once with 200 µl/well of PBS-T, 50 µl of 1% BSA/PBS-T was added and incubated on a shaking platform for 1 h at RT. After the solution was removed, diluted serum was added at 50 µl/well and incubated while shaking for 18 h at 4°C. The plate was then washed 3 times with PBS-T and tap dried. Then anti-mouse IgG or IgM antibodies conjugated to HRP (1:10,000 in PBS-T with 5% FCS and 1% BSA) was added at 50 µl/well, and incubated with shaking for 3 h at RT. After washing 3 times with PBS-T, the plate was tap dried. TMB solution was added at 50 µl/well for 15 min at RT in the dark and the absorbance read at 630 nm using a microplate reader.

A total of 36 plasma samples were collected and grouped into three sets according to the date of harvest (day 7, 18 or 25). Briefly, mice in Group 1 were sacrificed after one immunisation, Group 2 after two immunisations and finally Group 3 after three immunisations with either 20 µg of asparaginase or PMPC-asparaginase (20 µg of asparaginase), and their plasma collected. Table 2 shows the sample IDs used for the individual plasma samples.

| Table 2: Plasma sample IDs and their respective harvest dates | | | | | | | | |
|---|---|---|---|---|---|---|---|---|
| Group 1 (day 7) | | | Group 2 (day 18) | | | Group 3 (day 25) | | |
| Asp | MPC-Asp | PBS | Asp | MPC-Asp | PBS | Asp | MPC-Asp | PBS |
| A1-1 | P1-1 | Q1-1 | A2-1 | P2-1 | Q2-1 | A3-1 | P3-1 | Q3-1 |
| A1-2 | P1-2 | Q1-2 | A2-2 | P2-2 | Q2-2 | A3-2 | P3-2 | Q3-2 |
| A1-3 | P1-3 | | A2-3 | P2-3 | | A3-3 | P3-3 | |
| A1-4 | P1-4 | | A2-4 | P2-4 | | A3-4 | P3-4 | |
| A1-5 | P1-5 | | A2-5 | P2-5 | | A3-5 | P3-5 | |

*Quantitative titre of α-Asp IgG in plasma samples using asparaginase-coated ELISA*

Firstly, the titres of α-Asp IgG in the plasma samples from the three Groups were measured in ELISA plates coated with 100 ng of asparaginase (Asp-coated ELISA). The plasma was appropriately diluted and added to the coated wells. The amount of α-Asp IgG was then measured by the addition of α-mouse secondary antibody whose titre was subsequently measured colorimetrically at 630 nm. By testing a known dilution of commercially available α-Asp IgG (AbD Serotec), a standard curve could be plotted to obtain the relationship between antibody concentration and the absorbance. The absorbance readings were therefore converted into quantitative titres (ng/ml of antibody) using their respective IgG standard curve.

The α-Asp IgG titres within each group were then combined and pooled in order to obtain a mean antibody titre per Group (Figure 5). The mean values are shown in Table 3. According to the pooled results, there was a slight increase in α-Asp IgG titre on day 7 in Asp inoculated mice (25 ± 3 ng/ml) compared to the MPC-Asp-inoculated mice (16 ± 2 ng/ml). By day 18, there was a huge increase in the antibody titre in Asp-inoculated mice (4749 ± 1160 ng/ml) due to the highly immunogenic nature of the native asparaginase. By comparison, there was no significant increase in the antibody titre in mice inoculated with MPC-Asp (25 ± 5 ng/ml). By day 25, α-Asp IgG titre was raised even higher (8379 ± 2993 ng/ml) in Asp-inoculated mice whereas there was only a minor lift observed in PMPC-Asp-inoculated mice (123 ± 42 ng/ml). This is therefore evidence supporting the protective effect of PMPC polymer shielding the antigenic sites of asparaginase.

| Table 3: Mean α-Asp IgG titre pooled from five mice inoculated with asparaginase (Asp), PMPC-asparaginase (PMPC-Asp) or PBS in Asp-coated ELISA. n represents the number of values. Antibody titre unit is ng/ml. | | | |
|---|---|---|---|
| Day | Asp-inoculated | MPC-Asp inoculated | PBS-inoculated |
| 7 | 25 ±3 (n=30) | 16 ± 2 (n=30) | 13 ± 2 (n=19) |
| 18 | 4749 ± 1160 (n=30) | 25 ± 5 (n=30) | 9 ± 2 (n=12) |
| 25 | 8379 ± 2993 (n=30) | 123 ± 42 (n=29*) | 9 ± 1 (n=12) |
| *Some values were below the level of detection and were not included in the analysis | | | |

Figure 5 shows the expected immunogenic response for asparaginase. There is a manifold increase in α-Asp IgG titre by day 18 (day 7 is too early to observe a substantial amount of antigen-specific IgG) and this level is maintained by day 25 after the second booster on day 18. The IgG level will eventually diminish in the absence of antigenic challenge, but this level is expected to continue in the short run. For MPC-asparaginase-inoculated mice, there is a gradual increase in the α-Asp IgG titre. This is likely due to the shielding of the antigenic epitopes on the surface of asparaginase by the conjugated MPC polymer. The difference between the two immunogenic responses within 25 days is staggering, and illustrates the role of the MPC polymer in lowering immunogenicity of the conjugated protein.

## Claims

1. Use of a polymer comprising 2-methacryloyloxyethyl-phosphoryl choline (MPC) for reducing the *in vivo* immunogenicity, exhibited by lower antibody titre, of a therapeutic protein which is to be administered to the human or animal body, wherein the polymer is conjugated to the therapeutic protein via a covalent link.

2. The use according to claim 1, wherein the monomers consist substantially only of MPC monomers.

3. The use according to claim 1, wherein the protein comprises at least one cysteine residue and the protein is covalently linked to the polymer via one or more of said cysteine residues.

4. The use according to claim 1, wherein the covalently linked conjugate of polymer and therapeutic protein is of formula I or II: 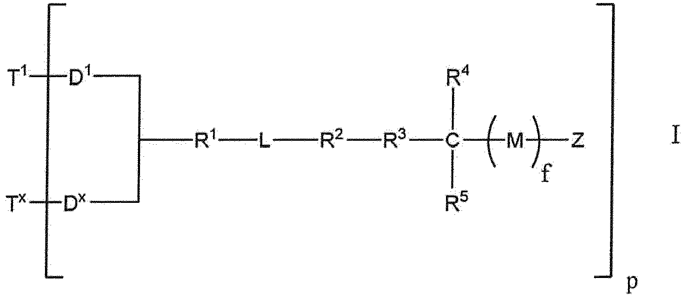 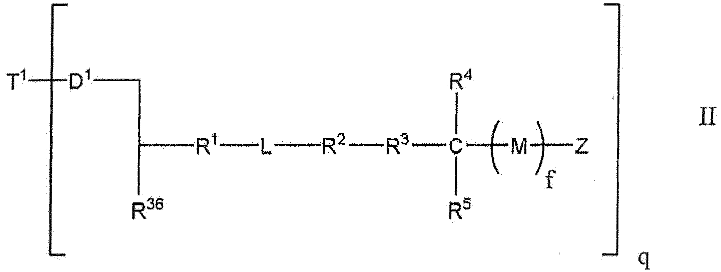
wherein R¹ is an electron-withdrawing group;
R² is selected from C=O, C(=O)NR⁹ or a bond; wherein R⁹ is H or C₁₋₄ alkyl;
R³ is selected from the group consisting of C₁-C₂₀ alkylene, C₃-C₈ cycloalkylene, C(=O)R¹⁰, C(=O)NR¹¹, C₁-C₂₀ alkoxy, C₂-C₂₀ alkenylene, C₂-C₂₀ alkenyl oxiranylene, arylene, heterocyclene and aralkylene; in which 0 to all of the hydrogen atoms are replaced with halogen, C₁-C₆ alkyl optionally substituted with 1 to 2 substituents selected from the group consisting of C₁-C₄ alkoxy, aryl, heterocyclyl, C(=O)R¹³, C(=O)NR¹¹R¹², oxiranyl and glycidyl;
R¹⁰ is alkylene of from 1 to 20 carbon atoms, alkoxy from 1 to 20 carbon atoms, oligo(alkoxy) in which each alkoxy group has from 1 to 3 carbon atoms, aryloxy or heterocyclyloxy; any of which groups may have substituents selected from optionally substituted alkoxy, oligoalkoxy, amino (including mono- and dialkyl amino and trialkyl ammonium, which alkyl groups, in turn, may have substituents selected from acyl, alkoxycarbonyl, alkenoxycarbonyl, aryl and hydroxy) and hydroxyl groups;
R¹¹ and R¹² are independently H or alkyl of from 1 to 20 carbon atoms, or R¹¹ and R¹² may be joined together to form an alkanediyl group of from 2 to 5 carbon atoms, thus forming a 3- to 6- membered ring;
R⁴ and R⁵ are each independently selected from H, Z, halogen, C₁₋₂₀ alkyl, C₃-C₈ cycloalkyl, OH, CN, C₂-C₂₀ alkenyl, C₂-C₂₀ alkenyl oxiranyl, C(=O)R¹³, glycidyl, aryl, heterocyclyl, arylkyl, aralkenyl, in which 0 to all of the hydrogen atoms are replaced with halogen, C₁-C₆ alkyl optionally substituted with 1 to 2 substituents selected from the group consisting of C₁-C₄ alkoxy, aryl, heterocyclyl, C(=O)R¹³, C(=O)NR¹¹R¹², oxiranyl and glycidyl;
where R¹³ is alkyl of from 1 to 20 carbon atoms, alkoxy of from 1 to 20 carbon atoms, oligo(alkoxy) in which each alkoxy group has 1 to 3 carbon atoms, aryloxy or heterocyclyloxy any of which groups may have substituents selected from optionally substituted alkoxy, oligoalkoxy, amino (including mono and di-alkyl amino and trialkyl ammonium, which alkyl groups, in turn may have substituents selected from acyl, alkoxycarbonyl, alkenoxycarbonyl, aryl and hydroxy) and hydroxyl groups; and L is a linking group;
Groups M are the same or different and are residues of ethylenically unsaturated monomers, wherein at least one of the residues M is MPC;
f is 1 to 500;
R³⁶ is -CH₂E⁴, H or C₁₋₄ alkyl; and
E⁴ is a leaving group;
x is 1 or 2;
p is 1-5 and q is 1-10;
D is S, NH or O; and
T-D is a therapeutic protein; wherein T¹D¹ and T²D² (if present) are different biological molecules.

5. The use according to claim 4, wherein each M is a residue of MPC.

6. The use according to claim 4 or 5, wherein R³ is CO-R¹⁰ in which R¹⁰ is oligoalkoxy, preferably oligoethoxy in which there are 2 to 10 ethoxy groups.

7. The use according to any of claims 4-6, wherein R¹ is selected from a keto group, an ester group and a sulphone group.

8. The use according to any of claims 4-7, wherein the linking group L, is selected from a bond, a C₁₋₁₀ alkylene group, or an optionally substituted aryl or heteroaryl, any of which groups may have substituents selected from optionally substituted alkoxy, oligoalkoxy, amino (including mono- and di-alkyl amino and trialkyl ammonium, which alkyl groups, in turn, may have substituents selected from acyl, alkoxycarbonyl, alkenoxycarbonyl, aryl and hydroxy) and
hydroxyl groups.

9. The use according to any of claims 4-8, wherein the polymer is of formula

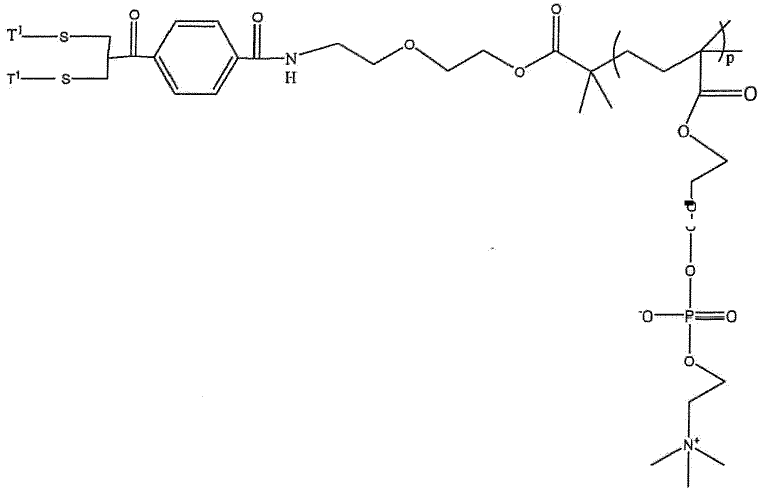

10. The use according to any preceding claim, wherein the therapeutic protein is an enzyme.

11. The use according to claim 10, wherein the enzyme is selected from asparaginase, arginase, adenosine deaminase, superoxide dismutase, catalase, chymotrypsin, lipase, uricase, bilirubin oxidase, glucose oxidase, glucuronidase, galactosidase, glucocerbrosidase, and glutaminase.

12. The use according to claim 10, wherein the therapeutic protein is asparaginase.

## Patentansprüche

1. Verwendung eines Polymers, umfassend 2-Methacryloyloxyethylphosphorylcholin (MPC) zum Verringern der *In*-*vivo*-Immunogenität, verdeutlicht durch einen niedrigeren Antikörpertiter, eines therapeutischen Proteins, das an den menschlichen oder tierischen Körper zu verabreichen ist, wobei das Polymer über eine kovalente Kopplung an das therapeutische Protein konjugiert ist.

2. Verwendung nach Anspruch 1, wobei die Momomere im Wesentlichen nur aus MPC-Monomeren bestehen.

3. Verwendung nach Anspruch 1, wobei das Protein zumindest einen Cysteinrest umfasst und das Protein über einen oder mehrere von den Cysteinresten kovalent an das Polymer gekoppelt ist.

4. Verwendung nach Anspruch 1, wobei das kovalent gekoppelte Konjugat von Polymer und therapeutischem Protein die Formel I oder II aufweist: 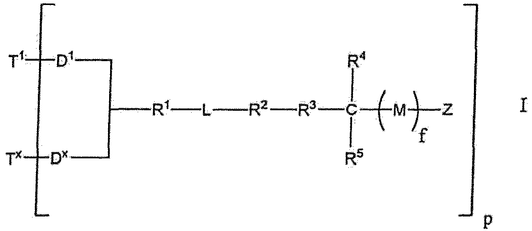 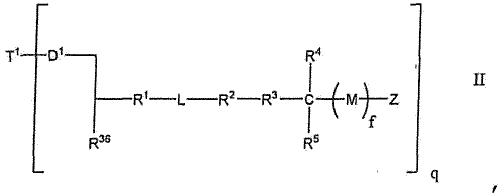
wobei R¹ eine elektronenziehende Gruppe ist;
R² aus C=O, C(=O)NR⁹ oder einer Bindung ausgewählt ist; wobei R⁹ für H oder C₁₋₄-Alkyl steht;
R³ aus der Gruppe ausgewählt ist, bestehend aus C₁-C₂₀-Alkylen, C₃-C₈-Cycloalkylen, C(=O)R¹⁰, C(=O)NR¹¹, C₁-C₂₀-Alkoxy, C₂-C₂₀-Alkenylen, C₂-C₂₀-Alkenyloxiranylen, Arylen, Heterocyclen und Aralkylen; wobei 0 bis alle der Wasserstoffatome mit Halogen ersetzt sind, C₁-C₆-Alkyl, optional substituiert mit 1 bis 2 Substituenten, ausgewählt aus der Gruppe, die aus C₁-C₄-Alkoxy, Aryl, Heterocyclyl, C(=O)R¹³, C(=O)NR¹¹R¹², Oxiranyl und Glycidyl besteht;
R¹⁰ für Alkylen aus von 1 bis 20 Kohlenstoffatomen, Alkoxy aus von 1 bis 20 Kohlenstoffatomen, Oligo(alkoxy), in dem jede Alkoxygruppe von 1 bis 3 Kohlenstoffatomen besitzt, Aryloxy oder Heterocyclyloxy steht; wobei eine jede dieser Gruppen Substituenten haben kann, die ausgewählt sind aus optional substituiertem Alkoxy, Oligoalkoxy, Amino (einschließlich Mono- und Dialkylamino und Trialkylammonium, wobei diese Alkylgruppen wiederum Substituenten haben können, die aus Acyl, Alkoxycarbonyl, Alkenoxycarbonyl, Aryl und Hydroxy ausgewählt sind) und Hydroxylgruppen;
R¹¹ und R¹² jeweils unabhängig für H oder Alkyl aus von 1 bis 20 Kohlenstoffatomen stehen, oder R¹¹ und R¹² zusammengefügt sein können, um eine Alkandiylgruppe aus von 2 bis 5 Kohlenstoffatomen zu bilden, wodurch ein 3- bis 6-gliedriger Ring entsteht;
R⁴ und R⁵ jeweils unabhängig ausgewählt sind aus H, Z, Halogen, C₁₋₂₀-Alkyl, C₃-C₈-Cycloalkyl, OH, CN, C₂-C₂₀-Alkenyl, C₂-C₂₀-Alkenyloxiranyl, C(=O)R¹³, Glycidyl, Aryl, Heterocyclyl, Arylkyl, Aralkenyl, wobei 0 bis alle der Wasserstoffatome ersetzt sind mit Halogen, C₁-C₆-Alkyl, optional substituiert mit 1 bis 2 Substituenten, ausgewählt aus der Gruppe, die aus C₁-C₄-Alkoxy, Aryl, Heterocyclyl, C(=O)R¹³, C(=O)NR¹¹R¹², Oxiranyl und Glycidyl besteht;
wobei R¹³ ist: Alkyl aus von 1 bis 20 Kohlenstoffatomen, Alkoxy aus von 1 bis 20 Kohlenstoffatomen, Oligo(alkoxy), wobei jede Alkoxygruppe 1 bis 3 Kohlenstoffatome besitzt, Aryloxy oder Heterocyclyloxy, wobei eine jede dieser Gruppen Substituenten haben kann, ausgewählt aus optional substituiertem Alkoxy, Oligoalkoxy, Amino (einschließlich Mono- und Dialkylamino und Trialkylammonium, wobei die Alkygruppen wiederum Substituenten haben können, die aus Acyl, Alkoxycarbonyl, Alkenoxycarbonyl, Aryl und Hydroxy ausgewählt sind) und Hydroxylgruppen; und L eine Kopplungsgruppe ist;
wobei die M-Gruppen gleich oder verschiedenartig sind und Reste von ethylenisch ungesättigten Monomeren sind, wobei es sich bei wenigstens einem der M-Reste um MPC handelt;
f für 1 bis 500 steht;
R³⁶ für -CH₂E⁴, H oder C₁₋₄-Alkyl steht; und
E⁴ eine Austrittsgruppe ist;
x für 1 oder 2 steht;
p für 1-5 steht und q für 1-10 steht;
D für S, NH oder O steht; und
T-D ein therapeutisches Protein ist; wobei T¹D¹ und T²D² (falls vorhanden) für verschiedene biologische Moleküle stehen.

5. Verwendung nach Anspruch 4, wobei jedes M ein Rest von MPC ist.

6. Verwendung nach Anspruch 4 oder 5, wobei R³ für CO-R¹⁰ steht, wobei R¹⁰ Oligoalkoxy, vorzugsweise Oligoethoxy ist, worin 2 bis 10 Ethoxygruppen vorhanden sind.

7. Verwendung nach einem der Ansprüche 4-6, wobei R¹ aus einer Ketogruppe, einer Estergruppe und einer Sulfongruppe ausgewählt ist.

8. Verwendung nach einem der Ansprüche 4-7, wobei die Kopplungsgruppe L ausgewählt ist aus einer Bindung, einer C₁₋₁₀-Alkylengruppe oder einem optional substituiertem Aryl oder Heteroaryl, wobei eine jede dieser Gruppen Substituenten haben kann, ausgewählt aus optional substituiertem Alkoxy, Oligoalkoxy, Amino (einschließlich Mono- und Dialkylamino und Trialkylammonium, wobei diese Alkygruppen wiederum Substituenten haben können, die aus Acyl, Alkoxycarbonyl, Alkenoxycarbonyl, Aryl und Hydroxy ausgewählt sind) und Hydroxylgruppen.

9. Verwendung nach einem der Ansprüche 4-8, wobei das Polymer die Formel 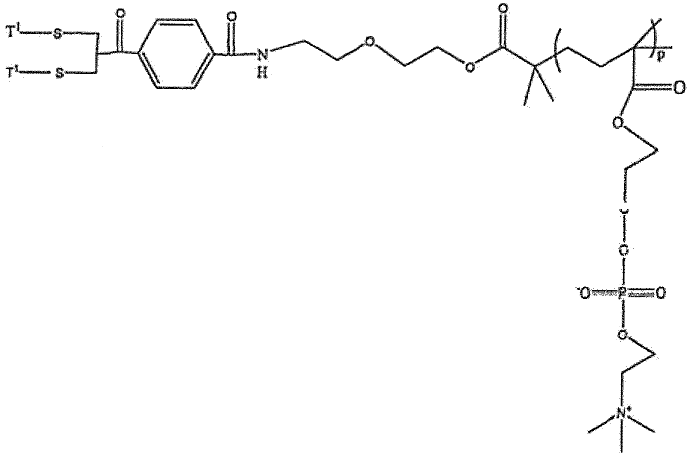 aufweist.

10. Verwendung nach einem vorhergehenden Anspruch, wobei es sich bei dem therapeutischen Protein um ein Enzym handelt.

11. Verwendung nach Anspruch 10, wobei das Enzym ausgewählt ist aus Asparaginase, Arginase, Adenosin-Deaminase, Superoxiddismutase, Catalase, Chymotrypsin, Lipase, Uricase, Bilirubinoxidase, Glucoseoxidase, Glururonidase, Galactosidase, Glucocerebrosidase und Glutaminase.

12. Verwendung nach Anspruch 10, wobei es sich bei dem therapeutischen Protein um Asparaginase handelt.

## Revendications

1. Utilisation d'un polymère comprenant de la 2-méthacryloyloxyéthyl-phosphorylcholine (MPC) pour réduire l'immunogénicité *in vivo,* présentée par un titre d'anticorps plus bas, d'une protéine thérapeutique qui est destinée à être administrée à l'organisme humain ou animal, dans laquelle le polymère est conjugué à la protéine thérapeutique via une liaison covalente.

2. Utilisation selon la revendication 1, dans laquelle les monomères sont sensiblement constitués uniquement par des monomères de MPC.

3. Utilisation selon la revendication 1, dans laquelle la protéine comprend au moins un résidu cystéine et la protéine est liée de manière covalente au polymère via un ou plusieurs desdits résidus cystéine.

4. Utilisation selon la revendication 1, dans laquelle le conjugué lié de manière covalente de polymère et de protéine thérapeutique répond à la formule I ou II : 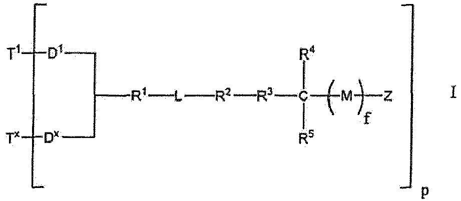 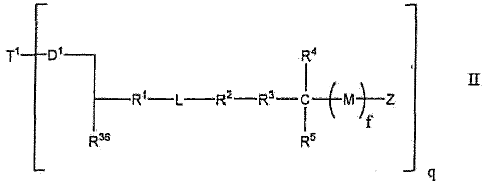 dans laquelle R¹ est un groupe attracteur d'électrons :
R² est sélectionné à partir de C=O, C(=O)NR⁹ ou d'une liaison ; dans laquelle R⁹ est H ou un alkyle en C₁₋₄ ;
R³ est sélectionné à partir du groupe constitué par un alkylène en C₁-C₂₀, un cycloalkylène en C₃-C₈, C(=O)R¹⁰, C(=O)NR¹¹, un alcoxy en C₁-C₂₀, un alcénylène en C₂-C₂₀, un oxyranylène alcényle en C₂-C₂₀, un arylène, un hétérocyclène et un aralkylène ; dans lesquels 0 à tous les atomes d'hydrogène sont remplacés par un halogène, un alkyle en C₁-C₆ facultativement substitué par 1 à 2 substituants sélectionnés à partir du groupe constitué par un alcoxy en C₁-C₄, un aryle, un hétérocyclyle, C(=O)R¹³, C(=O)NR¹¹R¹², un oxyranyle et un glycidyle ;
R¹⁰ est un alkylène de 1 à 20 atomes de carbone, un alcoxy de 1 à 20 atomes de carbone, un oligo(alcoxy) dans lequel chaque groupe alcoxy a de 1 à 3 atomes de carbone, un aryloxy ou un hétéroaryloxy ; dont l'un quelconque de ces groupes peut avoir des substituants sélectionnés à partir d'un alcoxy facultativement substitué, d'un oligoalcoxy, d'un amino (y compris le mono- et le dialkylamino et le trialkylammonium, lesquels groupes alkyle, à leur tour, peuvent avoir des substituants sélectionnés à partir d'un acyle, d'un alcoxycarbonyle, d'un alcénoxycarbonyle, d'un aryle et d'un hydroxyle) et de groupes hydroxyle ;
R¹¹ et R¹² sont indépendamment H ou un alkyle de 1 à 20 atomes de carbone, ou R¹¹ et R¹² peuvent être réunis ensemble pour former un groupe alcanediyle de 2 à 5 atomes de carbone, formant ainsi un cycle à 3 à 6 chaînons ;
R⁴ et R⁵ sont chacun sélectionnés indépendamment à partir de H, Z, d'un halogène, d'un alkyle en C₁₋₂₀, d'un cycloalkyle en C₃-C₈, OH, CN, d'un alcényle en C₂-C₂₀, d'un oxyranyle alcényle en C₂-C₂₀, C(=O)R¹³, d'un glycidyle, d'un aryle, d'un hétérocyclyle, d'un arylkyle, d'un aralcényle, dans lesquels 0 à tous les atomes d'hydrogène sont remplacés par un halogène, un alkyle en C₁-C₆ facultativement substitué par 1 à 2 substituants sélectionnés à partir du groupe constitué par un alcoxy en C₁-C₄, un aryle, un hétérocyclyle, C(=O)R¹³, C(=O)NR¹¹R¹², un oxyranyle et un glycidyle ;
où R¹³ est un alkyle de 1 à 20 atomes de carbone, un alcoxy de 1 à 20 atomes de carbone, un oligo(alcoxy) dans lequel chaque groupe alcoxy a 1 à 3 atomes de carbone, un aryloxy ou un hétérocyclyloxy dont l'un quelconque de ces groupes peut avoir des substituants sélectionnés à partir d'un alcoxy facultativement substitué, d'un oligoalcoxy, d'un amino (y compris le mono- et le dialkylamino et le trialkylammonium, lesquels groupes alkyle, à leur tour, peuvent avoir des substituants sélectionnés à partir d'un acyle, d'un alcoxycarbonyle, d'un alcénoxycarbonyle, d'un aryle et d'un hydroxyle) et de groupes hydroxyle ; et L est un groupe de liaison ;
les groupes M sont identiques ou différents et sont des résidus de monomères éthyléniquement insaturés,
dans laquelle au moins l'un des résidus M est la MPC ;
f vaut de 1 à 500 ;
R³⁶ est -CH₂E⁴, H ou un alkyle en C₁₋₄ ; et
E⁴ est un groupe partant ;
X vaut 1 ou 2 ;
P vaut 1 à 5 et q vaut 1 à 10 ;
D est S, NH ou O ; et
T-D est une protéine thérapeutique ; dans laquelle T¹D¹ et T²D² (le cas échéant) sont des molécules biologiques différentes.

5. Utilisation selon la revendication 4, dans laquelle chaque M est un résidu de MPC.

6. Utilisation selon la revendication 4 ou 5, dans laquelle R³ est CO-R¹⁰ dans lequel R¹⁰ est un oligoalcoxy, de préférence un oligoéthoxy dans lequel il y a 2à 10 groupes éthoxy.

7. Utilisation selon l'une quelconque des revendications 4 à 6, dans laquelle R¹ est sélectionné à partir d'un groupe céto, d'un groupe ester et d'un groupe sulfone.

8. Utilisation selon l'une quelconque des revendications 4 à 7, dans laquelle le groupe de liaison L est sélectionné à partir d'une liaison, d'un groupe alkylène en C₁₋₁₀, ou d'un aryle ou d'un hétéroaryle facultativement substitué, dont l'un quelconque de ces groupes peut avoir des substituants sélectionnés à partir d'un alcoxy, d'un oligoalcoxy, d'un amino (y compris le mono- et le dialkylamino et le trialkylammonium, lesquels groupes alkyle, à leur tour, peuvent avoir des substituants sélectionnés à partir d'un acyle, d'un alcoxycarbonyle, d'un alcénoxycarbonyle, d'un aryle et d'un hydroxyle) et de groupes hydroxyle.

9. Utilisation selon l'une quelconque des revendications 4 à 8, dans laquelle le polymère répond à la formule

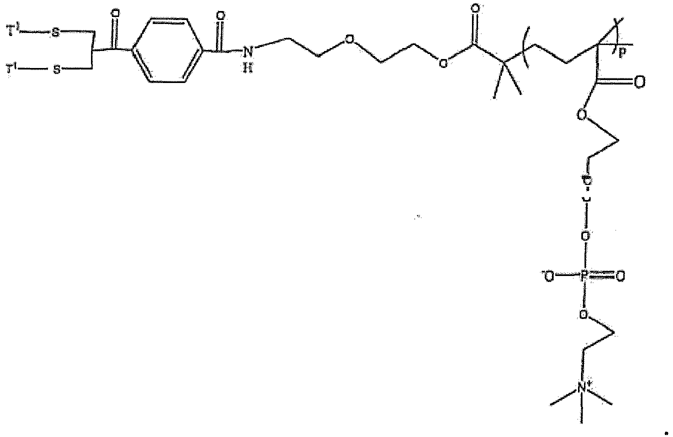

10. Utilisation selon une quelconque revendication précédente, dans laquelle la protéine thérapeutique est une enzyme.

11. Utilisation selon la revendication 10, dans laquelle l'enzyme est sélectionnée à partir de l'asparaginase, l'arginase, l'adénosine désaminase, la superoxyde dismutase, la catalase, la chymotrypsine, la lipase, l'uricase, la bilirubine oxydase, la glucose oxydase, la glucuronidase, la galactosidase, la glucocerbrosidase et la glutaminase.

12. Utilisation selon la revendication 10, dans laquelle la protéine thérapeutique est l'asparaginase.
